# EUROPEAN PATENT SPECIFICATION

(11) **EP 2 790 588 B1**
(45) Date of publication and mention of the grant of the patent: **13.11.2019**
(21) Application number: 12816350.8
(22) Date of filing: 06.12.2012
(51) Int. Cl.: A61B 8/06, G01N 29/00, G01S 15/00

(54) **AUTOMATED DOPPLER PULSE CYCLE SELECTION**
AUTOMATISIERTE DOPPLERIMPULS-ZYKLUSAUSWAHL
SÉLECTION AUTOMATIQUE DE CYCLES D'IMPULSIONS DOPPLER

(30) Priority: 16.12.2011 US 201161576630 P
(43) Date of publication of application: 22.10.2014
(73) Proprietor: Koninklijke Philips N.V., 5656 AE Eindhoven (NL)
(72) Inventor: GUPTA, Lalit, NL-5656 AE Eindhoven (NL); VAJINEPALLI, Pallavi, NL-5656 AE Eindhoven (NL); SISODIA, Rajendra Singh, NL-5656 AE Eindhoven (NL); RAMACHANDRAN, Ganesan, NL-5656 AE Eindhoven (NL); FIRTION, Celine, NL-5656 AE Eindhoven (NL); PETRUZZELLO, John, NL-5656 AE Eindhoven (NL); ANAND, Ajay, NL-5656 AE Eindhoven (NL)
(74) Representative: Steffen, Thomas
(86) International application number: PCT/IB2012/057033
(87) International publication number: WO 2013/088314

(56) References cited:
- WO-A2-2009/013686
- US-A- 5 628 321
- US-A1- 2008 300 486
- US-A1- 2009 326 379
- US-A1- 2010 234 731
- PALLAVI V ET AL: "Doppler based identification of uterine artery and umbilical artery for monitoring pregnancy", 2010 ANNUAL INTERNATIONAL CONFERENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, 31 August 2010 (2010-08-31), pages 6300-6303, XP032109713, DOI: 10.1109/IEMBS.2010.5628089 ISBN: 978-1-4244-4123-5
- CLOUTIER G ET AL: "The Effect Of Averaging Cardiac Doppler Spectrograms On Their Amplitude Variability", ENGINEERING IN MEDICINE AND BIOLOGY SOCIETY, 1990., PROCEEDINGS OF THE TWELFTH ANNUAL INTERNATIONAL CONFERENCE OF THE IEEE PHILADELPHIA, PA, USA 1-4 NOV. 1990, NEW YORK, NY, USA,IEEE, US, 1990, pages 560-561, XP010035878, DOI: 10.1109/IEMBS.1990.691214 ISBN: 978-0-87942-559-3

## Description

### FIELD OF THE INVENTION

The present invention relates to pulsatile flow and, more particularly, to a device and a computer readable medium for selecting pulse cycles representative of the flow.

### BACKGROUND OF THE INVENTION

Commercial duplex ultrasound systems are used extensively to localize blood vessels and obtain flow characteristics from the blood vessels. For example, in obstetrics, applications exist for uterine arteries, the umbilical artery, the mid cerebral artery and in cardiac applications, the carotid artery and so on. A duplex ultrasound system combines the modality of real-time, two dimensional, pulse-echo imaging of anatomical structures with that of a Doppler ultrasound system from which the Doppler frequency shift or the velocity information is obtainable from a blood vessel.

The use of ultrasound in vascular applications to perform Doppler velocimetry requires the accurate computation of flow parameters to produce consistent, reproducible and reliable diagnosis.

The accuracy with which flow parameters are computed is dependent on the cycles chosen by a sonologist or doctor, with good cycles being selected manually.

Doppler exams typically require a great degree of skill to obtain a clinically useful measurement. For example, correct orientation of the probe with respect to the vessel is essential to ensure that the beam-flow angle is less than 60 degrees. Errors in measurements are amplified when angles of greater than 60 degrees are used in the determination of velocities. The standard workflow on a clinical ultrasound scanner allows a sonographer to determine the orientation of the probe with respect to the vessel using a standard B-mode and Color Flow display. The spectral Doppler measurements are then obtained thus ensuring that the measured velocities are correct.

The use of ultrasound in vascular applications to perform Doppler velocimetry requires availability of skilled personnel.

In emerging market countries such as India, the shortage of specialists limits the availability and access to ultrasound. Hence, an automated method of acquiring and evaluating Doppler signals for clinical diagnosis (without requiring the user to interpret an ultrasound scan image) would be useful to non-radiologists such as OB/GYN or cardiologists who are the primary treatment providers.

In addition, a low-cost system is essential to provide an attractive solution in emerging market environments. Devices that are currently available on the market for antenatal check-ups and labor are the ultrasound scanner and the fetal monitor/cardiotoco graph (CTG) machine. However, both of these devices are relatively expensive.

There exists a need for a low-cost, easy-to-use solution to provide Doppler velocimetry to screen for and monitor high risk pregnancies.

In addition, even with duplex ultrasound systems and likewise in medical applications other than obstetrics, the manual, i.e., visual, selection of good pulse cycles requires specialized skill and is a tedious and time consuming task. In particular, a good cycle is one which represents the actual hemodynamic profile in a vessel.

Also, the judgment as to what constitutes a good cycle varies among observers.

Especially in emerging markets such as India, automatic selection is needed.

Commonly-assigned patent applications WO 2012/085788 A2 entitled "Automated Doppler Velocimetry Using a Low-Cost Transducer" and and WO 2013/042029 A1 entitled "Excitation Schemes for Low-Cost Transducer Arrays" disclose a hand-held, stand-alone, Doppler-based, ultrasound probe whose examining face is less finely divided into separate transducer elements, i.e., for relatively few separate elements. As mentioned therein, the probe operates automatically without the need for interpreting a visual display of anatomy.

In the conference proceedings paper "Doppler based identification of uterine artery and umbilical artery for monitoring pregnancy", by V. Pallavi et al., 2010 Annual International Conference of the IEEE Engineering in Medicine and Biology Society (EMBC 2010), Buenos Aires, Argentina, 31 August - 4 September 2010, IEEE, Piscataway, NJ, USA, 31 August 2010, pp. 6300-6303 (XP032109713), it is there disclosed an algorithm to identify umbilical and uterine arteries from a set of four different maternal and fetal arteries using their Doppler signatures. In order to distinguish these arteries, spectrograms of a plurality of Doppler signals collected from pregnant women are generated to extract good cycles, which are then analyzed to derive independent features that could uniquely represent an artery. A non-linear classification technique using k-NN (k-nearest neighbor) classifier is further applied to identify umbilical and uterine arteries.

### SUMMARY OF THE INVENTION

The invention is defined by the claims.

The present invention is directed to novel, automatic pulse cycle selection, with particular application to the probe referred to immediately above and to user-interactive-imaging systems such as duplex ultrasound systems.

In accordance with an aspect of the present invention, a device is configured for examining pulsatile flow, for deriving, based on the examined flow, a plurality of spectral characteristics and for, based on the derived characteristics, determining which one or more pulse cycles in a spectrogram are to be selected as representative of said flow, said cycles subject to the selection respectively having a plurality of specified parameters, said determining excluding from said selection a cycle, from among said cycles, if any of said specified parameters of said cycle deviates, by more than a predetermined amount, from a respective average which the device derives from said spectrogram.

In accordance with a sub-aspect, the selected cycles are consecutive.

In accordance with another sub-aspect, the selecting chooses a predetermined number of cycles. That number can be five.

In accordance with one other sub-aspect, the device includes a display and is further configured for, responsive to the determining, displaying a selected cycle or selected cycles.

According to a different sub-aspect, the device is further configured for operating on a selected cycle to compute a clinical parameter. The clinical parameters are typically computed on each of the selected five cycles and an average value from these five cycles is taken as clinical parameter value.

In accordance with a further sub-aspect, the device includes a display and is further configured for, responsive to the computing, displaying the clinical parameter.

In accordance with a related sub-aspect, the flow is that of a blood vessel.

According to a complementary sub-aspect, the examining includes receiving ultrasound. The device is further configured for generating, from the received ultrasound, the cycles subject to selection.

In a yet another sub-aspect, a handheld, stand-alone, diagnostic apparatus incorporates the device.

In a related sub-aspect, the device features transducer elements and is configured not to collectively use any of these elements to focus, nor to steer, any beam used for the examining to perform the deriving.

In accordance with an additional sub-aspect, the device includes a user interface for specifying a blood flow parameter, for use in the determining, and/or a medical application for which the determining is performed.

According to one other additional sub-aspect, the device includes a user interface comprising a display. The device is further configured for making the selection by choosing a plurality of segments that each are made up of multiple ones of the cycles and for, on the display, displaying the segments for user selection via the user interface.

According to a further, supplementary sub-aspect, the device is further configured for distinguishing the displayed segments by highlighting them.

In a yet further sub-aspect, the average is a median.

As a particular sub-aspect, a cycle having a plurality of specified parameters, the device is further configured for: a) by specified parameter, using as an exponent the absolute value of the deviation of the parameter from an average to form a term; and b) summing the terms over the plurality of parameters to yield a cycle quality metric.

In a further sub-aspect, the base for said exponent is a function of the base of the natural logarithm.

Alternatively or in addition, in a further sub-aspect, the determining includes summing the metrics to yield a cycle-series segment quality metric.

In a yet different sub-aspect, an example of a characteristic is a Doppler-spectral-waveform caliper measurement on a cycle. The determining entails computing a deviation of the measurement.

In yet one other sub-aspect, a selected cycle is representative of frequency shift versus time.

In yet an additional sub-aspect, the device is configured for, based on at least one of a group of conditions, filtering out a cycle of said cycles subject to the selection, said group consisting of: a) existence in said cycle of at least one of more than one peak and more than one valley; b) frequency response in a spectral band of said cycle not exceeding a predetermined threshold; c) said cycle not reaching its peak systolic value within a predetermined duration; and d) said cycle having less than a predetermined length.

In accordance with another aspect of the present invention, a computer readable medium for selecting pulse cycles, said medium comprising a computer program having instructions executable by a processor to cause the device of the previous aspect to perform a plurality of acts, among said plurality there being the acts of:
deriving and determining that the aforementioned device performs.

Details of the novel, automated pulse cycle selection device and the computer readable medium for selecting pulse cycles are set forth further below, with the aid of the following drawings, which are not drawn to scale.

### BRIEF DESCRIPTION OF THE DRAWINGS

FIG. 1 is a schematic diagram showing, by example, an ultrasound probe, a volume of interest containing blood vessels, and a blood-flow waveform and respective clinical Doppler indices;
FIG. 2 is a conceptual diagram exemplifying a good pulse cycle and a cycle-series segment quality metric;
FIG. 3 is a schematic diagram of one possible visual display of segments of pulse cycles, the segments serving as candidates for selection; and
FIG. 4 is a flow chart exemplary of a process for selecting good pulse cycles.

### DETAILED DESCRIPTION OF EMBODIMENTS

The description of what is proposed herein with regard to automatic pulse cycle selection is preceded with what largely is a review of the Doppler-based probe disclosed in the commonly-assigned patent applications mentioned herein above.

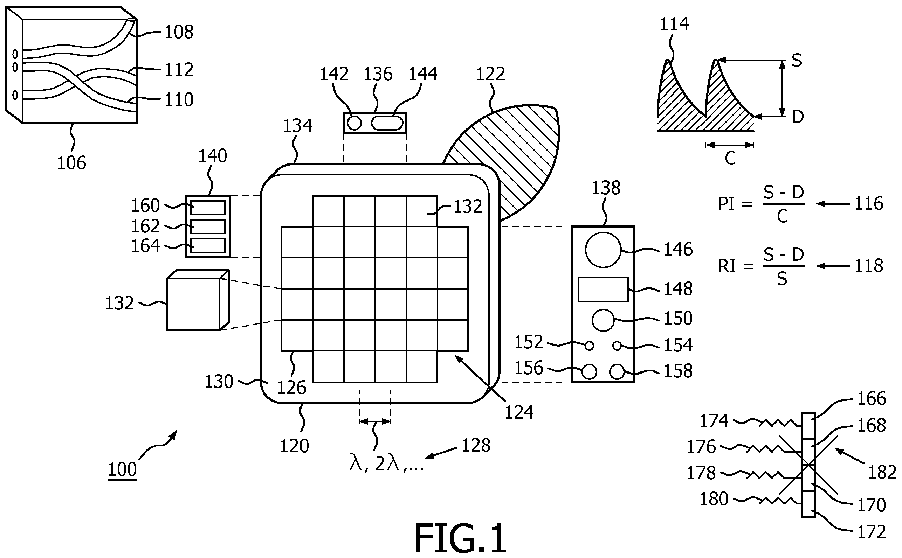

FIG. 1 depicts, by way of illustrative and non-limitative example, an ultrasound probe 100 and a volume or "volume of interest" 106 containing blood vessels 108, 110, 112. Further depicted are a blood-flow, or "spectral Doppler ultrasound", waveform 114, i.e., envelope of a spectrogram, and respective clinical Doppler indices 116, 118.

The probe 100 is implementable as an automatic, handheld, stand-alone, self-contained, ultrasound examination device. It has a transducer housing 120 and a handle 122.

Within the transducer housing 120, a non-phased, two-dimensional transducer array 124 is comprised of transducer elements 126, the number of elements being determined by the scan volume and anatomy. Data acquisition occurs individually by element 126, although, as discussed in more detail further below, elements are operable concurrently to shorten the total acquisition time period.

As seen in FIG. 1 by way example, the number of elements 126 is 32. Thus, with an element size of 10 mm, an approximately 6 cm x 6 cm volume is covered. Flush with a front surface 130 of the housing 120, are ultrasound-receiving faces 132 of the transducer elements 126, the same faces also transmitting, i.e., issuing, ultrasound.

The total of merely 32 elements 126 stands in stark contrast to the much greater number of elements that would be required in conventional medical imaging to cover the same 6 cm x 6 cm volume.

In this regard, electronic focusing for medical imaging, as with a phased-array transducer, requires an inter-element spacing of a ½ wavelength, i.e., ½ λ, or less. Doppler ultrasound for imaging can typically range from between 2 x 10⁶ and 4 x 10⁶ cycles per second (2 to 4 MHz). Ultrasound travels through soft body tissue at a speed of about 1540 meters/second. Wavelength, i.e., λ, is equal to velocity divided by frequency. Here, this is 1540 m/s divided by approximately 2 x 10⁶ cycle/s = 0.8 millimeter. Medical ultrasound imaging for a display would therefore require an inter-element spacing of less than 0.4 mm, and an element surface area of less than (0.4 mm)² which is less than 0.2 mm². Therefore, with a small element size on the order of ½ λ, thousands of elements 126 would be required to build a 2D array that, like the one seen in FIG. 1, covers a volume of 6 cm by 6 cm.

The spacing (size) of elements in FIG. 1 is 10 mm, which, as discussed above, would ordinarily be more than 12λ of ultrasound used in examining the volume of interest 106 for the blood vessels 108, 110, 112 present.

More generally, the elements 126, in accordance with what is proposed herein, are spaced apart by more than ½λ, although inter-element spacing 128 may be λ, 2λ or more, as discussed hereinabove. The area of the face 132 is, correspondingly, at least 0.6 square millimeters (mm²), and may be more, such as 10 mm², 25 mm², or 100 mm² as in FIG. 1.

Advantageously, the automatic ultrasound device 100 does not rely on display of medical images to reach a diagnosis; but, instead, features an array composed of fewer transducer elements and therefore fewer channels. Thus, cost of production is low, while, by virtue of automatic operation, reliability is maintained. Reliability may even be improved, as when medical examinations must be performed at a quicker pace. The automatic operation also tends to reduce examination time, thereby relieving workload, and making the examination more convenient.

During Doppler data acquisition, the elements 126 are fired either sequentially, or in one or more groups taking care that the acoustic signal from one element does not significantly affect others that are excited at the same time. For each element 126, the receive period lags the transmit period. The Doppler receive gate is correspondingly positioned in the receive period so as to enable sampling from a corresponding depth within the volume of interest 106.

On a back surface 134 of the housing 120, so as to face the user, are a number of user-interface, input-output panels which include a top panel 136, a left panel 138 and a right panel 140. An on-off switch 142 and an audio speaker face 144 are disposed in the top panel 136. The left panel 138 frames a function navigation/actuation button 146, a display 148, a Doppler power detection indicator 150, fetal heartbeat acquisition indicator 152, a maternal heartbeat acquisition indicator 154, a normal blood-flow indicator 156, and an abnormal blood-flow indicator 158. The right panel 140 includes three initializing-parameter-entry feedback windows 160, 162, 164.

The elements 126 of the array 124 all are operated to image independently.

This stands in contrast to phased arrays for example, which use multiple separate transducer elements collectively to image or steer a beam. In phased arrays, the steering and focusing is performed by appropriately delaying the input and/or output of elements with respect to other elements.

In accordance with what is proposed herein, the transducer elements of a group are fired simultaneously. The group elements continue imaging concurrently, and independently by element, until expiration of the group's data acquisition time period.

A device for the imaging by groups is configured not to collectively use any of the elements 126 to focus, nor to steer, a beam used in the imaging. By way of demonstration, the transducer elements 166, 168, 170, 172 in FIG. 1 each have their respective signals 174, 176, 178, 180. The signals 174, 180 on transmit lag the transmission signals 176, 178 thereby resulting in focus and/or steering of a resultant ultrasound beam. The probe 100 is not implemented for such a protocol, as indicated by the "X" 182 in FIG. 1. Likewise, on receive, no delay is differentially applied to the elements 166, 168, 170, 172.

The blood-flow waveform 114 is a graph of Doppler frequency shift versus time and is thereby indicative of blood flow velocity versus time.

Clinical Doppler indices, such as the pulsatility index (PI) 116 and the resistance index (RI) 118 are Doppler angle-independent measures of blood pulsatility. The symbols S, D and C annotating the blood-flow waveform 114 represent, respectively, the peak systolic frequency shift, the end diastolic frequency shift, and the length of one cardiac cycle. Another commonly-used clinical Doppler index is the systolic/diastolic ratio S/D. The symbols S, D and C are Doppler-spectral-waveform caliper measurements. These and other clinical, or blood-flow, parameters such as the clinical Doppler indices are examples of spectral characteristics of pulsatile flow, based upon which the quality of a cycle can be judged. Another spectral characteristic is the frequency response within a spectral band, which if sufficiently low indicates lack of quality of the cycle. Likewise, if a cycle does not achieve its peak systolic value, or its complete cycle length, within a given time period, it is deemed to lack quality.

The probe 100 can utilize the above-identified Doppler indices in identifying blood vessels and in assessing normality of blood flow.

The signal processing involved in classifying, and analyzing, a blood vessel 108-112 found by the probe 100 in the volume of interest 106 and more details on the probe and its use are disclosed in the above-mentioned commonly-assigned patent applications.

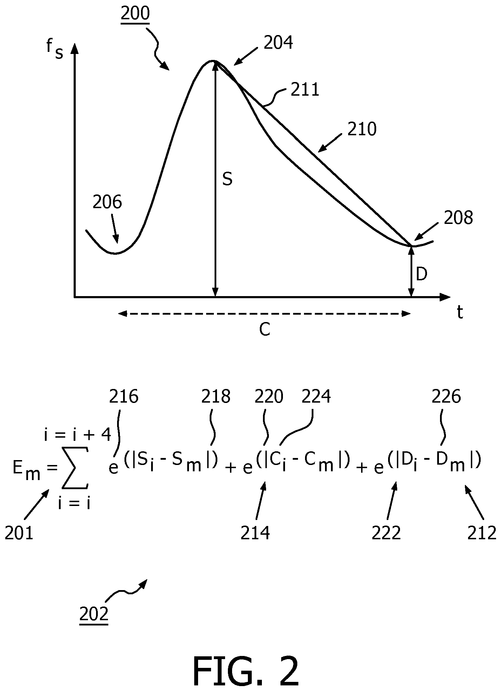

FIG. 2 exemplifies a good pulse cycle 200 in a spectrogram of a Doppler signal, and a cycle-series segment quality metric 202 for selecting a series of good pulse cycles from a spectrogram. The Doppler signal has been extracted from the carrier frequency, i.e., that of the pulse to be echoed from the blood flow. The extraction is made using a quadrature demodulator. Output from the demodulator is bandpass filtered to remove low frequencies signals that may originate from slow moving structures such as vessel walls and soft tissue, and signals having frequencies above a certain level. The resulting Doppler signal is indicative of the Doppler frequency shift, and of the blood flow velocity.

A typical good pulse cycle 200, corresponding to a single heart beat, has a significant peak 204 between two valleys 206, 208 within an acceptable time duration since the beginning of the cycle. The acceptable time duration varies in maternal and fetal arteries. The time duration of a maternal artery could vary from 0.6 sec to 1.5 sec and for fetal arteries it could be from 0.3 sec to 1.0 sec. It is also checked that there are no local peaks 204 or valleys 208. Thus, each pulse cycle 200 preferably has a single peak 204 and a single valley 208.

Since the rise and fall in frequency shift during the cycle 200 respectively represent rise and fall in blood flow velocity, a good cycle will exhibit a smooth rise and a smooth fall. The fall part, i.e., from peak to valley, of the cycle 200 undergoes another test. It is checked whether a line 210 joining the peak 204 and the valley 208 that immediately follows the peak crosses the waveform 114 at least once. A crossing point 211 is shown in FIG. 2. Lack of such a crossing point 211 indicates that the cycle 200 is not sufficiently representative of the pulsatile blood flow.

Also, some of the spectral information may not be continuously interpretable, as the targeted blood vessel 108-112 may not be within the sample volume due to motion of the patient or sonologist, or the motion of the blood vessel 108-112 itself. Some of the pulse cycles 200 may not be of good quality for interpretation either if the ultrasound probe 100 does not make proper angle with the blood vessel 108-112 or due to noise from neighboring tissue or in-built electronics.

As a result, portions of the spectrogram may be weak, and seen on screen as faded or missing, indicating that associated frequency samples of the Fast Fourier Transform (FFT) that processes the incoming Doppler signal are low in magnitude. Other types of anomalies that are observed in the shape of the spectrogram profile include weak peak strength, incomplete cycle 200, absence of peak 204 and sharp tall peak.

Based on these considerations, some of the cycles 200 are initially filtered out. Spectral characteristics of the surviving cycles 200 are extracted and used in scoring segments of five consecutive, surviving cycles. Relying on five cycles is an acceptable clinical practice. The present inventors have empirically found it prudent to select a series of consecutive cycles 200. The cycle-series segment quality metric 202, shown as Eₘ in FIG. 2 is used for the scoring. It is the sum 201 of five cycle quality metrics 212, one for each of five consecutive cycles 200. Each cycle quality metric 212 consists of three terms 214, although additional terms can be added for medical applications other than obstetrics. Each term 214 has a base 216 and an exponent 218. The base 216 is a function of what is commonly referred to as "e", the base of the natural logarithm (In). In the example of FIG. 2, the function is the identity function. The base 216 can be any value above unity, such as "e" or any larger number. The exponent 218 is the absolute value 220 of a deviation 222. The deviation 222 is between a Doppler-spectral-waveform caliper measurement 224 on a cycle 200 and a median 226 of that measurement over the cycles of the spectrogram that have not been excluded as anomalous. The symbols Sᵢ, Cᵢ, and Dᵢ represent, the systole, cycle length and diastole of cycle i. The exponents 218 are the respective absolute values 220 of the deviations 222 of the systole, cycle length and diastole from the corresponding medians 226. The median is used as a measure of representative value, because the median is robust to extreme observations and yet serves as a type of average. The exponential nature of the terms 214 ensures that the cost of even a single cycle 200 deviating from the median 226 incurs a high penalty. The equation for Eₘ is extendable to include other terms 214 for other medical applications. For instance, a characteristic for the carotid artery is spectral broadening, which is the distance between the peak in the maximum frequency envelope and the peak in the minimum frequency envelope. The maximum frequency envelope is the envelope applied to the spectrogram with respect to the maximum frequencies in blood flow. In particular, due to its viscosity, blood cross-sectionally flows through an artery at different speeds, lagging near the periphery adjacent the artery walls. For cardiac applications such as those relating to the mitral valve, the tricuspid valve and the aortic valve, the characteristics include the isovolume relaxation time, the isovolume acceleration time, the acceleration time in early systole, and the deceleration time in early diastole. In renal applications, spectral broadening of the renal artery can serve as a characteristic.

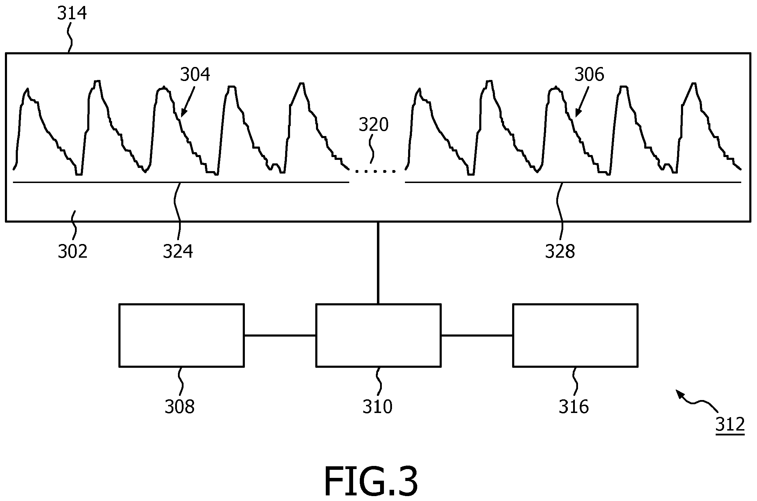

FIG. 3 depicts an implementation that includes a visual display 302 of segments 304, 306 of pulse cycles 200, the segments serving as candidates for selection by the user after having been scored according to Eₘ. An ultrasound probe 308 in this implementation can, but need not, be designed as a standalone device. The probe 308 is, wirelessly or by wire, connectable or connected to a processor 310 which, in turn, is connected to a user interface 312. The user interface 312 has a display screen 314 and user actuatable controls 316. A touch-sensitive feature for the screen 314 can be included among the controls 316, as can other navigation and selection devices such as a mouse, buttons, keys slides, knobs and trackballs.

The display 302 continuously shifts across the display screen 314, as from left to right. Any segment 304, 306 currently on screen is selectable, e.g., by touching the touch screen. In the rolling display, the dotted line 320 between the two segments 304, 306 represents temporally where cycles 200, or high scoring (and therefore less desirable) segments, have been excluded. The displayed segments 304, 306 are highlighted, for example by brightness, color, or, as shown here, underlining 324, 328. Along with the highlighting, each segment 304, 306 maybe accompanied in its translation across the screen 314 with caliper measurements 224 for each cycle 200 and/or segment scores. When the segment 304, 306 is selected, Doppler parameters 116, 118 for the cycles 200 in the segment are computed and appear on screen.

As an alternative to the rolling segments, the system can automatically select the segment or segment(s) 304, 306 with the lowest scores, compute the respective parameters 116, 118 and display the selected segments and parameters.

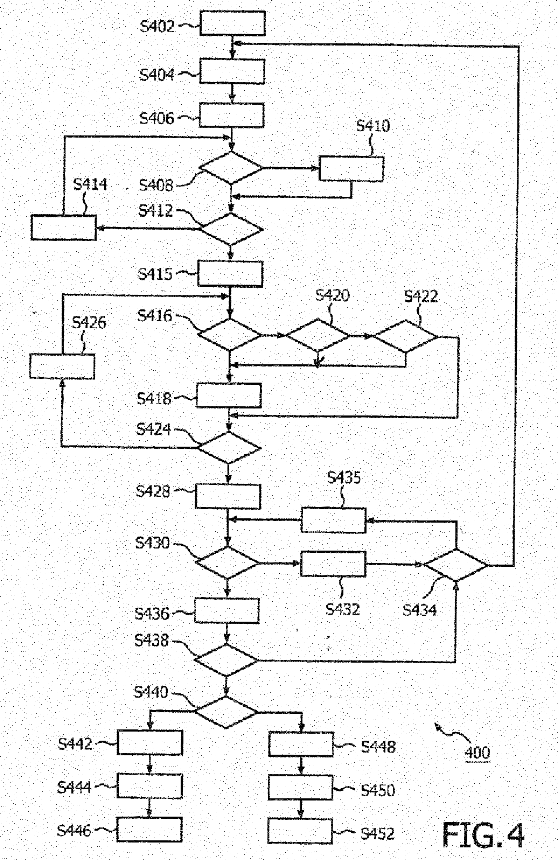

FIG. 4 provides, by way of illustrative and non-limitative example, a process 400 for selecting good pulse cycles. The user or operator, who may be a clinician, midwife, general practitioner, obstetrician/gynecologist or fetal radiologist, selects a medical application, such as obstetrics, or one or more parameters from a set of parameters (step S402). A Doppler signal is captured during a brief period of time from the received ultrasound (step S404). A spectrogram is computed (step S406).

If sufficient frequency response is lacking over some spectral band of the current cycle 200 under consideration, as judged from a power threshold (step S408), the current cycle is excluded from selection (step S410).

In either case, if there are more cycles 200 from the data that has been captured in step S404 (step S412), the next cycle is processed (step S414), and processing returns to step S408 with that next cycle serving as the current cycle.

When there are no more cycles 200 to consider (step S412), the cycles that have not been excluded collectively constitue a spectrogram having gaps where cycles have been excluded. Thus, the gapped spectrogram typically has one or more time portions in which cycles have not been excluded. An envelope is, by any known and suitable method, computed for the spectrogram time portion(s) that individually comprise five or more consecutive cycles, and thus have the potential for furnishing a segment of five, consecutive, good cycles 200 (step S415). A method for extracting an envelope from a spectrogram is described in the United States Patent No. 7,611,467 to Zhang.

Cycle filtering continues. For example, caliper measurements 224 are made for the current cycle 200, both as to its length of time and the time duration until its peak.

If either measurement 224 exceeds respective a predetermined range of normality (S416), the current cycle 200 is excluded (step S418). Otherwise, if neither range is exceeded, query is made as to whether more than one peak exists in the current cycle (step S420), and as to whether more than one valley exists in the current cycle (step S422). For either condition, the current cycle is excluded (step S418).

In any event, if more cycles are available for consideration (step S424), processing returns to step S416 with the next cycle serving as the current cycle (step S426).

When there are no more cycles for the above-described filtering procedure (step S424), the median 226 is calculated over all surviving cycles. This done for each parameter, such a caliper measurement 224, selected directly or by implication is step S402 (step S428). The calculation is an initial step in computing Eₘ, the cycle-series segment quality metric 202, shown in FIG. 2.

For a current segment 304, 306 of five, consecutive ones of the cycles 200 remaining from the above-described filtering, query is made on the deviation 222 for each cycle of the segment. In particular, query is made as to whether the absolute value 220 of the deviation 222 of a parameter from its median 226 exceeds a predetermined deviation threshold. This query is made for each of the selected parameters (step S430). The thresholds may be set so as to detect deviations of 25% or more from the respective median 226. If the deviation threshold is exceeded (step S430), thereby indicating that the current cycle is unworkable, processing shifts cycle-wise past the deviant cycle 200 (step S432).

If, despite the shift, five or more of the cycles 200 that have survived filtering before step S424 are currently available for forming a segment (S434), return is made to step S430 with the next segment 304, 306 serving as the current segment (S435).

If, on the other hand, fewer than five cycles 200 are available (S434), return is made to step S404.

If the deviation threshold is not exceeded for the current segment 304, 306 (step S430), the segment is scored to yield the cycle-series segment quality metric 202 (step S436).

If more segments 304, 306 are desired (step S438), processing branches back to step S434.

Otherwise, if no further scored, good segments 304, 306 are desired (step S438), and the user is to select from among these segments (step S440), the segments are displayed, and highlighted, for selection as described herein above (step S442). The segments 304, 306 are optionally accompanied on screen by caliper measurements 224 and segment scores. Parameters such as Doppler indices are then computed for the segment 304, 306 selected by the user (step S444), and both the segment and indices are displayed on screen 314 (step S446). If, on the othe other hand, no further scored, good segments 304, 306 are desired (step S438), and the selection is automatic (step S440), the segment with the minimum score is selected (step S448). Likewise, parameters are then computed for the selected segment 304, 306 (step S450), and the segment and parameters are displayed (step S452). The computation may involve averaging results for the five cycles 200 of the selected segment 304, 306, with the average being included on the display screen 314.

By virtue of a caliper measurement, operation on a cycle from among the cycles 200 selected is performed to compute a clinical parameter, this all being done automatically in the innnovative technique. More generally, the computation typically involves such measurements on each cycle 200, and averaging of results.

Within the probe 100, 308, control circuitry (not shown), serving as the device proposed herein, can take the form of one or more integrated circuits (ICs). The one or more ICs can, alternatively, be configured for installation into existing apparatus such as ultrasound Duplex scanners.

A signal for operating the device, i.e., IC(s), probe or duplex system, in accordance with the techniques proposed herein can be formed internally, formed by varying an electrical current applied to a wire input to the device, or applied to an antenna for wireless transmission of the signal and reception by a receiving antenna of the device.

A device is configured for examining pulsatile flow, for deriving, based on the examined flow, spectral characteristics and for, based on the derived characteristics, determining which one or more pulse cycles are to be selected as representative of the flow. The cycles selected can be consecutive and amount to a predetermined number of cycles, such as five. The cycles subject to selection may initially be filtered out based on waveform anomalies, with the surviving cycles in a consecutive group of sufficient number being judged based on parameters such as waveform caliper measurements and other types of the characteristics. Good cycles are detected by their lack of variation, with respect to the measured parameters, from each respective, parameter median over the spectrogram cycles not initially filtered. The technique may, according to user selection, take into account additional parameters suited to particular medical application. Uses include correctly identifying an artery by name.

Although methodology of the present invention can advantageously be applied in providing medical diagnosis for a human or animal subject, the scope of the present invention is not so limited. More broadly, techniques disclosed herein are directed to efficiently finding, and subjecting to improved fluid-flow analysis, vessels in body tissue, in vivo, in vitro or ex vivo.

What is proposed herein pertains to selecting good cycles of spectral Doppler waveforms, the selected cycles being representative of blood flow, for rendering a clinical diagnosis based on a result of analyzing the selected cycles. The technique is particularly useful for accurately identifying an artery by name as in the commonly-owned patent application entitled "Automatic Blood Vessel Identification by Name" by the same inventors. Applications of the technology proposed herein include carotid and renal arteries screening, anke-brachial index (ABI) measurements for detecting peripheral arterial disease (PAD), transcranial and cardiac examinations, bleed detection in trauma or other hemorrhages, in addition to fetal well-being assessment.

While the invention has been illustrated and described in detail in the drawings and foregoing description, such illustration and description are to be considered illustrative or exemplary and not restrictive; the invention is not limited to the disclosed embodiments.

For example, waveform anomalies relating to the shape of a cycle peak can be detected and used in the filtering out of cycles, e.g., step S418.

A computer program can be stored momentarily, temporarily or for a longer period of time on a suitable computer-readable medium, such as an optical storage medium or a solid-state medium. Such a medium is non-transitory only in the sense of not being a transitory, propagating signal, but includes other forms of computer-readable media such as register memory, processor cache and RAM.

A single processor or other unit may fulfill the functions of several items recited in the claims. The mere fact that certain measures are recited in mutually different dependent claims does not indicate that a combination of these measures cannot be used to advantage.

## Claims

1. A device (308) configured for examining pulsatile flow (S404), for deriving, based on the examined flow, a plurality of spectral characteristics and for, based on the derived characteristics, determining which one or more pulse cycles in a spectrogram (114) are to be selected as representative of said flow, said cycles subject to the selection respectively having a plurality of specified parameters, said determining excluding from said selection a cycle, from among said cycles, if any of said specified parameters of said cycle deviates (222), by more than a predetermined amount, from a respective average which the device derives from said spectrogram.

2. The device of claim 1, the selected cycles (200) being consecutive.

3. The device of claim 1, the selecting choosing a predetermined number of cycles.

4. The device of claim 3, said number being five.

5. The device of claim 1, comprising a display (314) and further configured for, responsive to said determining, displaying a cycle from among the cycles that have been selected.

6. The device of claim 1, further configured for operating on a cycle, from among the cycles that have been selected, to compute a clinical parameter.

7. The device of claim 6, comprising a display and further configured for, responsive to the computing, displaying said clinical parameter (116, 118).

8. The device of claim 1, comprising a user interface that includes a display, said device being further configured for making the selection by choosing a plurality of segments (304, 306) that each are made up of multiple ones of said cycles and for, on said display, displaying said segments for, via said interface, user selection of a segment from among said segments.

9. The device of claim 8, further configured for distinguishing the displayed segments by highlighting them (324, 328).

10. The device of claim 1, said average being a median (226).

11. The device of claim 1, further configured for filtering out a candidate cycle, from among said cycles subject to the selection, if frequency response in a spectral band of said candidate cycle does not exceed a predetermined threshold.

12. A computer readable medium for selecting pulse cycles, said medium comprising a computer program having instructions executable by a processor to cause the device of any of claims 1 to 11 to perform a plurality of acts, among said plurality there being the acts of:
deriving and determining as specified in claim 1.

## Patentansprüche

1. Vorrichtung (308), konfiguriert zum Untersuchen einer pulsierenden Strömung (S404), zum Ableiten, basierend auf der untersuchten Strömung, einer Vielzahl von Spektraleigenschaften und zum Bestimmen, basierend auf den abgeleiteten Eigenschaften, welcher eine oder mehrere Impulszyklen in einem Spektrogramm (114) als repräsentativ für die Strömung ausgewählt werden sollen, wobei die Zyklen, die der Auswahl unterliegen, jeweils eine Vielzahl von vorgegebenen Parametern aufweisen, wobei das Bestimmen einen Zyklus von der Auswahl der Zyklen ausschließt, wenn einer der spezifizierten Parameter des Zyklus um mehr als einen vorbestimmten Betrag von einem entsprechenden Mittelwert abweicht (222), den die Vorrichtung aus dem Spektrogramm ableitet.

2. Vorrichtung nach Anspruch 1, wobei die ausgewählten Zyklen (200) aufeinanderfolgend sind.

3. Vorrichtung nach Anspruch 1, wobei die Auswahl eine vorbestimmte Anzahl von Zyklen wählt.

4. Vorrichtung nach Anspruch 3, wobei die Zahl fünf ist.

5. Vorrichtung nach Anspruch 1, umfassend eine Anzeige (314) und weiter konfiguriert, um als Reaktion auf das Bestimmen einen Zyklus aus den ausgewählten Zyklen anzuzeigen.

6. Vorrichtung nach Anspruch 1, weiter konfiguriert, um in einem Zyklus aus den ausgewählten Zyklen zu arbeiten, um einen klinischen Parameter zu berechnen.

7. Vorrichtung nach Anspruch 6, umfassend eine Anzeige und weiter konfiguriert, um als Reaktion auf das Rechnen den klinischen Parameter (116, 118) anzuzeigen.

8. Vorrichtung nach Anspruch 1, umfassend eine Benutzerschnittstelle, die eine Anzeige beinhaltet, wobei die Vorrichtung weiter konfiguriert ist, um die Auswahl durch Auswählen einer Vielzahl von Segmenten (304, 306) zu treffen, die jeweils aus mehreren der Zyklen bestehen, und um auf der Anzeige die Segmente anzuzeigen, um über die Schnittstelle eine Benutzerauswahl eines Segments aus den Segmenten vorzunehmen.

9. Vorrichtung nach Anspruch 8, weiter konfiguriert zur Unterscheidung der angezeigten Segmente durch Hervorheben derselben (324, 328).

10. Vorrichtung nach Anspruch 1, wobei der Durchschnitt ein Median (226) ist.

11. Vorrichtung nach Anspruch 1, weiter konfiguriert, um einen Kandidatenzyklus aus den der Auswahl unterliegenden Zyklen herauszufiltern, wenn der Frequenzgang in einem Spektralband des Kandidatenzyklus einen vorbestimmten Schwellenwert nicht überschreitet.

12. Computerlesbares Medium zum Auswählen von Impulszyklen, wobei das Medium ein Computerprogramm mit Anweisungen umfasst, die von einem Prozessor ausgeführt werden können, um die Vorrichtung nach einem der Ansprüche 1 bis 11 zu veranlassen, eine Vielzahl von Handlungen auszuführen, wobei es unter der Vielzahl die Handlungen gibt von:
Ableiten und Bestimmen nach Anspruch 1.

## Revendications

1. Dispositif (308) configuré pour examiner un flux pulsé (S404), pour déduire, sur la base du flux examiné, une pluralité de caractéristiques spectrales et pour déterminer, sur la base des caractéristiques déduites, lesquels d'un ou plusieurs cycles d'impulsions dans un spectrogramme (114) doivent être sélectionnés comme représentatifs dudit flux, lesdits cycles soumis à la sélection ayant respectivement une pluralité de paramètres spécifiés, ladite détermination excluant de ladite sélection un cycle, parmi lesdits cycles, si l'un quelconque desdits paramètres spécifiés dudit cycle s'écarte (222), de plus d'une quantité prédéterminée, d'une moyenne respective que le dispositif déduit dudit spectrogramme.

2. Dispositif selon la revendication 1, les cycles sélectionnés (200) étant consécutifs.

3. Dispositif selon la revendication 1, la sélection choisissant un nombre prédéterminé de cycles.

4. Dispositif selon la revendication 3, ledit nombre étant cinq.

5. Dispositif selon la revendication 1, comprenant un affichage (314) et configuré en outre pour, en réponse à ladite détermination, afficher un cycle parmi les cycles qui ont été sélectionnés.

6. Dispositif selon la revendication 1, configuré en outre pour fonctionner sur un cycle, parmi les cycles sélectionnés, afin de calculer un paramètre clinique.

7. Dispositif de la revendication 6, comprenant un affichage et configuré en outre pour, en réponse au calcul, afficher ledit paramètre clinique (116, 118).

8. Dispositif selon la revendication 1, comprenant une interface utilisateur qui inclut un affichage, ledit dispositif étant en outre configuré pour effectuer la sélection en choisissant une pluralité de segments (304, 306) qui sont chacun composés de plusieurs desdits cycles et pour, sur ledit affichage, afficher lesdits segments pour, via ladite interface, une sélection par un utilisateur d'un segment parmi lesdits segments.

9. Dispositif selon la revendication 8, configuré en outre pour distinguer les segments affichés en les mettant en surbrillance (324, 328).

10. Dispositif selon la revendication 1, ladite moyenne étant une médiane (226).

11. Dispositif de la revendication 1, configuré en outre pour filtrer un cycle candidat parmi lesdits cycles soumis à la sélection, si la réponse en fréquence dans une bande spectrale dudit cycle candidat ne dépasse pas un seuil prédéterminé.

12. Support lisible par ordinateur pour sélectionner des cycles d'impulsions, ledit support comprenant un programme informatique comportant des instructions exécutables par un processeur pour amener le dispositif selon l'une quelconque des revendications 1 à 11 à exécuter une pluralité d'actes, et parmi ladite pluralité d'actes, les actes consistant à déduire et déterminer comme spécifié dans la revendication 1.
